# EUROPÄISCHE PATENTSCHRIFT

(11) **EP 1 926 514 B1**
(45) Veröffentlichungstag und Bekanntmachung des Hinweises auf die Patenterteilung: **03.12.2008**
(21) Anmeldenummer: 06775147.9
(22) Anmeldetag: 22.08.2006
(51) Int. Cl.: A61M 5/315, A61M 5/24

(54) **INJEKTIONSVORRICHTUNG MIT ZWEIWEGE-RUTSCHKUPPLUNG**
INJECTION DEVICE WITH TWO-WAY SLIP CLUTCH
DISPOSITIF D'INJECTION À ACCOUPLEMENT À GLISSEMENT À DEUX VOIES

(30) Priorität: 14.09.2005 DE 102005043806; 20.12.2005 DE 102005060929
(43) Veröffentlichungstag der Anmeldung: 04.06.2008
(73) Patentinhaber: TecPharma Licensing AG, 3401 Burgdorf (CH)
(72) Erfinder: BURREN, Stefan, CH-3047 Bremgarten (CH); KOHLBRENNER, Philippe, CH-3413 Kaltacker (CH); WITTMANN, Juergen, CH-3400 Burgdorf (CH); WITTWER, Martin, CH-3533 Bowil (CH)
(86) Internationale Anmeldenummer: PCT/CH2006/000453
(87) Internationale Veröffentlichungsnummer: WO 2007/030957

(56) Entgegenhaltungen:
- EP-B1- 1 351 732
- WO-A-01/72361
- DE-A1- 10 237 258
- US-A- 6 004 297

## Beschreibung

Die Erfindung betrifft eine Injektionsvorrichtung für ein injizierbares Produkt, vorzugsweise ein flüssiges Medikament wie beispielsweise Insulin, ein Wachstumshormon oder ein Osteoporosepräparat.

Aus der EP 1 351 732 B1 ist eine Injektionsvorrichtung bekannt mit einem Gehäuse, in dem ein Reservoir für ein injizierbares Produkt mit einem Förderkolben angeordnet sind. Eine Kolbenstange ist mit dem Gehäuse in einem Gewindeeingriff. Die Vorrichtung umfasst ferner ein Dosierglied, das in einem Gewindeeingriff mit der Kolbenstange relativ zu dem Gehäuse und der Kolbenstange axial beweglich ist, einen relativ zu dem Gehäuse drehbeweglichen, aber axial nicht beweglichen Betätigungsknopf, der mit dem Dosierglied verdrehgesichert verbunden ist, ein Kupplungsglied, das mit der Kolbenstange axial beweglich verdrehgesichert verbunden ist und mit dem Dosierglied eine Zweiwege-Rutschkupplung bildet, und schließlich ein Federglied, welches das Kupplungsglied mit einer Elastizitätskraft beaufschlagt und dadurch in einem Kupplungseingriff mit dem Dosierglied hält. Die Rutschkupplung ist als Drehrutschkupplung ausgebildet mit zwei Zahnkränzen, die miteinander in einem gegen die Kraft des Federglieds lösbaren Rasteingriff sind und so den Kupplungseingriff bilden. Der Rasteingriff ist gegen die Kraft des Federglieds in beide Drehrichtungen lösbar, so dass durch eine Drehbewegung des Dosierglieds in eine Dosierrichtung die Dosis vergrößert und durch eine Drehbewegung in eine entgegengesetzte Korrekturrichtung verkleinert werden kann. Das Federglied wirkt in Doppelfunktion als Kupplungsfeder, die das Kupplungsglied in den Kupplungseingriff mit dem Dosierglied spannt, und als Ausschüttfeder, deren Federspannung bei dem Einstellen durch Vergrößerung der Dosis erhöht wird und bei der Ausschüttung den Vortrieb des Kolbens bewirkt. Um bei der Einstellung der Dosis ein versehentliches Rutschen der Kupplung zu verhindern, müssen die im Kupplungseingriff befmdlichen Zahnkränze der Federspannung standhalten. Entsprechend groß ist der für die Vergrößerung der Dosis erforderliche Kraftaufwand des Benutzers.

Es ist eine Aufgabe der Erfindung, eine Injektionsvorrichtung zu schaffen, die ein sicheres Einstellen einer zu verabreichenden Dosis ohne besonderen Kraftaufwand einschließlich einer Dosiskorrektur erlaubt.

Die Erfindung betrifft eine Injektionsvorrichtung, die ein Gehäuse mit einem Reservoir für ein injizierbares Produkt, einen Betätigungsknopf für die Einstellung einer Pröduktdosis, ein Förderglied für die Ausschüttung der eingestellten Dosis, eine Zweiwege-Rutschkupplung und ein Federglied umfasst. Der Betätigungsknopf ist für die Einstellung der Dosis relativ zu dem Gehäuse in eine die Dosis vergrößernde Dosierrichtung und eine die Dosis verringernde Korrekturrichtung beweglich. Vorzugsweise ist der Betätigungsknopf relativ zu dem Gehäuse drehbeweglich. Obgleich weniger bevorzugt, kann der Betätigungsknopf für die Einstellung der Dosis relativ zu dem Gehäuse auch nur linear beweglich oder in noch einer alternativen Ausführung linear- und drehbeweglich sein. In bevorzugten einfachen Ausführungen bilden das Gehäuse und der Dosierknopf je ein Gelenkglied eines gemeinsam gebildeten Drehgelenks. Grundsätzlich kann der Dosierknopf jedoch auch erst über ein oder mehrere weitere Gelenkelemente und dementsprechend mehrere Gelenke mit dem Gehäuse beweglich verbunden sein. Zweckmäßigerweise kann ein in dem Reservoir linear beweglich aufgenommener Förderkolben das Förderglied bilden.

Die Zweiwege-Rutschkupplung umfasst wenigstens ein Rastelement und wenigstens ein Rastgegenelement, die in einem Rasteingriff miteinander verrastend den Dosierknopf bei einer Bewegung in die Dosierrichtung und bei einer Bewegung in die Korrekturrichtung jeweils in diskreten Rastpositionen mit dem Gehäuse form- und kraftschlüssig koppeln. Das Federglied wirkt der Bewegung des Dosierknopfs in wenigstens eine der beiden Richtungen, vorzugsweise der Bewegung in die Dosierrichtung, mit einer elastischen Federkraft entgegen.

Nach der Erfindung ist oder sind das Rastelement oder das Rastgegenelement in Abstimmung auf die Federkraft so geformt, dass sie im Rasteingriff der Bewegung in die durch die Federkraft begünstigte Richtung einen größeren Widerstand entgegensetzen als der Bewegung in die andere Richtung. Das Wort "oder" wird hier wie auch im übrigen im üblichen logischen Sinne von "und/oder" verwendet, soweit sich aus dem Zusammenhang des jeweiligen Gebrauchs des Worts kein anderer Sinn ergibt. Die Form des Rastelements oder des Rastgegenelements ist bevorzugt so gewählt, dass die Rutschkupplung der Bewegung des Betätigungsknopfs in die Korrekturrichtung einen größeren Widerstand als der Bewegung in die Dosierrichtung entgegensetzt und der für das Aufdosieren zu überwindende Reibwiderstand formbedingt geringer ist als der für die Dosiskorrektur zu überwindende Reibwiderstand.

In bevorzugter Ausführung wird das Federglied durch die Bewegung des Betätigungsknopfs in die wenigstens eine Richtung von Rastposition zu Rastposition stärker gespannt. Aufgrund des erfindungsgemäß geformten Rastelements oder Rastgegenelements kann ein versehentliches Durchrutschen der Kupplung in die andere Richtung, in die nämlich das Federglied wirkt, sicherer als im Stand der Technik verhindert werden, während gleichzeitig der durch die Form des wenigstens einen Rastelements oder Rastgegenelements bedingte Widerstand gegen die Bewegung in die wenigstens eine Richtung geringer als im Falle einer herkömmlichen, bezüglich der Bewegungsrichtung indifferenten Rutschkupplung sein kann. Aufgrund der Erfindung kann der für das Einstellen der Dosis erforderliche Kraftaufwand gegenüber dem Stand der Technik verringert und die Haltekraft der Kupplung dennoch vergrößert werden. Ebenso kann bei gleich bleibender Haltekraft der Kraftaufwand für die Einstellung der Dosis verringert oder bei gleich bleibendem Kraftaufwand die Haltekraft der Rutschkupplung vergrößert werden.

Das Federglied kann eine Kupplungsfeder bilden, gegen deren Federkraft das Rastelement und Rastgegenelement sowohl bei der Bewegung in die Dosierrichtung als auch bei der Bewegung in die Korrekturrichtung voneinander wegbewegt werden, um in der jeweils nächsten Rastposition wieder zu verrasten. Die Federkraft kann für beide Bewegungsrichtungen in jeder Rastposition die gleiche sein. Bevorzugter wird jedoch das Federglied bei der Bewegung in die wenigstens eine der Richtungen von Rastposition zu Rastposition stärker gespannt, so dass es der Bewegung in die betreffende Richtung eine größere Federkraft entgegensetzt als in die andere: Dabei kann es der Bewegung in die andere Richtung ebenfalls eine, wenn auch kleinere Federkraft entgegensetzen.

In bevorzugten Ausführungen setzt das Federglied nur der Bewegung in die wenigstens eine der beiden Richtungen eine Federkraft entgegen. In derartigen Ausführungen dient das Federglied nicht als Kupplungsfeder. Vorteilhafterweise ist in solchen Ausführungen eine Kupplungsfeder als weiteres Federglied oder sind mehrere Kupplungsfedern als weitere Federglieder vorgesehen, so dass das Rastelement und Rastgegenelement in beide Bewegungsrichtungen gegen die Elastizitätskraft der separaten Kupplungsfeder oder der mehreren separaten Kupplungsfedern von Rastposition zu Rastposition bewegbar sind. Die funktionale Entlastung des Federglieds in dieser Hinsicht eröffnet Gestaltungsspielraum hinsichtlich der Form und des Einbaus des Federglieds. Ferner kann das Federglied einfacher in einer anderen Zusatzfunktion verwendet werden. Desweiteren kann die Rutschkupplung mittels der wenigstens einen separaten Kupplungsfeder oder der mehreren separaten Kupplungsfedern im Hinblick auf ihre Haltekraft und den für die Überwindung der Haltekraft erforderlichen Kraftaufwand optimiert werden.

Das Federglied ist vorzugsweise eine mechanische Feder, kann alternativ aber auch eine pneumatische Feder sein. Der Funktion nach kann das Federglied insbesondere eine Druckfeder oder eine Torsionsfeder, gegebenenfalls auch eine Zugfeder sein. In bevorzugten Ausführungen ist das Förderglied bei der Ausschüttung der Dosis mittels des Federglieds antreibbar. In einer bevorzugten ersten Variante wird das Förderglied bei der Ausschüttung nur von dem Federglied angetrieben. In einer ebenfalls bevorzugten zweiten Variante muss der Benutzer die für den Antrieb des Förderglieds erforderliche Kraft zumindest zu einem Teil aufbringen, und das Förderglied erfüllt die Funktion einer Servofeder, die den Benutzer unterstützt, so dass sich bei der Ausschüttung der Benutzer und das Federglied die für den Antrieb des Förderglieds erforderliche Kraft teilen. Ein bevorzugtes Beispiel für die zweite Variante wird in der deutschen Patentanmeldung Nr. 10 2005 043 806.7 offenbart, die in Bezug genommen wird.

Das Rastelement und das Rastgegenelement sind von Rastposition zu Rastposition in eine Kupplungsdosierrichtung und eine Kupplungskorrekturrichtung bewegbar. Vorzugsweise sind die Kupplungsdosierrichtung und die Kupplungskorrekturrichtung einander entgegengerichtet. Vorteilhafterweise ist der Betätigungsknopf mit einem aus Rastelement und Rastgegenelement so verbunden, dass die Kupplungsdosierrichtung der Dosierrichtung und die Kupplungskorrekturrichtung der Korrekturrichtung des Betätigungsknopfs entsprechen. Vorzugsweise ist das eine aus Rastelement und Rastgegenelement an dem Betätigungsknopf geformt oder befestigt, so dass es dessen Bewegung mitmacht. Das eine aus Rastelement und Rastgegenelement kann mit dem Betätigungsknopf aber auch erst über ein oder mehrere Zwischenglieder kinematisch, d.h. schlupffrei, gekoppelt sein. In derartigen Ausführungen kann die Kupplungsdosierrichtung von der Dosierrichtung des Betätigungsknopfs abweichen. Entsprechendes gilt für die Kupplungskorrekturrichtung und die Korrekturrichtung des Betätigungsknopfs. In sämtlichen Ausführungen gilt jedoch, dass sich das Rastelement relativ zu dem Rastgegenelement in die Kupplungsdosierrichtung bewegt, wenn der Betätigungsknopf in die Dosierrichtung bewegt wird, und sich in die Kupplungskorrekturrichtung bewegt, wenn der Betätigungsknopf in die Korrekturrichtung bewegt wird.

Die Rutschkupplung umfasst wenigstens zwei Kupplungshälften, nämlich eine erste Kupplungshälfte mit dem wenigstens einen Rastelement und eine zweite Kupplungshälfte mit dem wenigstens einen Rastgegenelement. Das Rastelement und das Rastgegenelement sind an der jeweiligen Kupplungshälfte vorzugsweise in einem Stück angeformt, d.h. stoffschlüssig mit der zugeordneten Kupplungshälfte verbunden oder bevorzugter gleich bei der Urformung angeformt. Alternativ können sie an der jeweiligen Kupplungshälfte auch befestigt sein. In bevorzugten Ausführungen ist die Rutschkupplung eine Drehrutschkupplung, wobei eine der Kupplungshälften relativ zu der anderen zumindest für die Einstellung der Dosis um eine Drehachse drehbeweglich ist. Bevorzugt bildet das Gehäuse eine der Kupplungshälften. Ein separat gefertigtes, aber fest mit einer Gehäuseschale verbundenes Teil wird als zu dem Gehäuse gehörig gerechnet. Alternativ kann auch eine bezüglich der Drehachse verdrehgesichert mit dem Gehäuse, in anderer Weise jedoch relativ zu dem Gehäuse bewegliche Struktur, insbesondere eine axial bewegliche Struktur, eine der Kupplungshälften bilden. Grundsätzlich können sogar beide Kupplungshälften relativ zu dem Gehäuse um die Drehachse drehbeweglich sein, wobei allerdings eine der Kupplungshälften zumindest bei der Einstellung der Dosis verdrehgesichert mit dem Gehäuse verbunden oder gegebenenfalls über ein Getriebe mit dem Gehäuse gekoppelt ist. Falls eine mit dem Gehäuse verdrehgesichert verbundene, relativ zu dem Gehäuse jedoch axial bewegliche Struktur eine der Kupplungshälften bildet, wird es bevorzugt, wenn der Betätigungsknopf sich bei dem Einstellen der Dosis und auch bei dem Ausschütten gemeinsam mit der axial beweglichen Struktur in die axiale Richtung bewegt, wie dies insbesondere in der deutschen Patentanmeldung Nr. 10 2005 043 806.7 offenbart wird. Die Relativbewegung zwischen den Kupplungshälften kann im Grunde auch eine drehfrei Translationsbewegung oder eine aus Translation und Rotation zusammengesetzte Bewegung sein. Das vorstehend zur Drehrutschkupplung für die Kopplung einer der Kupplungshälften mit dem Gehäuse Gesagte gilt auch in Bezug auf derartige alternative Relativbewegungen zwischen den Kupplungshälften. So kann die Relativbewegung beispielsweise eine zu einer Längsachse der Injektionsvorrichtung parallele, axiale oder eine zu der Längsachse transversale Linearbewegung sein. Entsprechend kann das Gehäuse eine der Kupplungshälften, ein mit dem Gehäuse unbeweglich verbundenes Teil oder ein relativ zu dem Gehäuse in Richtung der Relativbeweglichkeit nicht bewegliches Teil die eine der Kupplungshälften bilden oder die eine der Kupplungshälften abstützen. Das vorstehend zur Drehrutschkupplung Gesagte gilt anstatt für die Relativdrehbewegung für die alternative Relativbeweglichkeit analog.

In bevorzugten Ausführungen umfasst die Rutschkupplung genau zwei Kupplungshälften, d.h. sie besteht in derartigen Ausführungen aus den genannten Kupplungshälften, und das Rastelement und das Rastgegenelement greifen im Rasteingriff ineinander. In hierzu alternativen Ausführungsformen umfasst die Rutschkupplung die zwei Kupplungshälften und ferner ein zwischen den Kupplungshälften angeordnetes Übertragungsglied. Das Übertragungsglied ist mit einer ersten der zwei Kupplungshälften in einem Rasteingriff und mit der zweiten der Kupplungshälften in einem weiteren Rasteingriff, vorzugsweise bildet es mit den beiden Kupplungshälften je eine Drehrutschkupplung. Eine derartige Rutschkupplung wird in der DE 102 37 258 A1 offenbart, die diesbezüglich in Bezug genommen wird. Die in solch alternativen Ausführungen dreigliedrige Rutschkupplung umfasst zwei Paare aus je wenigstens einem Rastelement und wenigstens einem Rastgegenelement. Die erste Kupplungshälfte ist relativ zu der zweiten Kupplungshälfte wieder in eine Kupplungsdosierrichtung und eine entgegengesetzte Kupplungskorrekturrichtung bewegbar, wobei sie im Rasteingriff mit dem Übertragungsglied relativ zu dem Übertragungsglied in eine dieser beiden Richtungen und im Rasteingriff des Übertragungsglieds mit der zweiten Kupplungshälfte relativ zu dieser nur gemeinsam mit dem Übertragungsglied in die andere Richtung bewegbar ist.

In der DE 102 37 258 A1 und auch in der deutschen Patentanmeldung Nr. 10 2005 043 806.7 greifen die Rastelemente und Rastgegenelemente der beiden Drehraster in Bezug auf die Drehachse axial ineinander. Alternativ können die Kupplungshälften und das dazwischen angeordnete Übertragungsglied jedoch auch einander umgeben, vorzugsweise koaxial, und die Rastelemente und Rastgegenelemente an den jeweils paarweise einander zugewandten Umfangsflächen geformt oder befestigt sein.

Das wenigstens eine Rastelement oder das wenigstens eine Rastgegenelement ist oder sind in bevorzugten Ausführungen in Bezug auf die beiden Richtungen, in welche sie relativ zueinander hin und her bewegbar sind, asymmetrisch geformt. Die Asymmetrie ist vorzugsweise derart, dass für die Bewegung des Betätigungsknopfs in die Korrekturrichtung eine größere Kraft aufgewendet werden muss als für die Bewegung in die Dosierrichtung. Der im Rasteingriff von Rastelement und Rastgegenelement zu überwindende Widerstand ist in die Korrekturrichtung somit größer als in die Dosierrichtung. Das Rastelement und Rastgegenelement, die einen Rastvorsprung und eine Rastvertiefung darstellen, weisen bezüglich den beiden Richtungen ihrer Relativbeweglichkeit Flanken auf, die in Bezug auf die beiden Richtungen asymmetrisch sind, indem eine in die eine der beiden Richtungen weisende Flanke des Rastelements oder des Rastgegenelements steiler ist als die in die andere Richtung weisende Flanke des gleichen Elements. Grundsätzlich genügt es, wenn nur das Rastelement oder nur das Rastgegenelement asymmetrisch geformt ist. Eine aufeinander angepasste, kongruente Form von Rastelement und Rastgegenelement wird jedoch bevorzugt.

Das Rastelement und das Rastgegenelement der Rutschkupplung, nämlich das Rastelement und Rastgegenelement der einander kontaktierenden Kupplungshälften in der einen Ausführung und die wenigstens zwei Paare aus Rastelement und Rastgegenelement der beiden Kupplungshälften und des Übertragungsglieds in der alternativen Ausführung, können an den Kupplungshälften und dem optionalen Übertragungsglied unnachgiebig angeordnet sein. In diesem Fall ist wenigstens eine der Kupplungshälften oder das optionale Übertragungsglied im Ganzen gegen eine Federkraft elastisch nachgiebig gelagert, damit die Rutschkupplung im Rasteingriff bewegt werden kann. In bevorzugten Ausführungen ist jedoch das Rastelement oder Rastgegenelement an der betreffenden Kupplungshälfte oder dem optionalen Übertragungsglied elastisch nachgiebig angeordnet. Insbesondere kann jedes Rastelement oder Rastgegenelement einen elastischen Schnapper bilden, indem pro Rastelement oder Rastgegenelement eine Kupplungsfeder vorgesehen ist, von welcher das zugehörige Rastelement abragt oder als Rastvertiefung ausgenommen ist. Obgleich sowohl das Rastelement als auch das Rastgegenelement je als elastischer Schnapper gebildet sein kann, wird es bevorzugt, wenn nur eines aus Rastelement und Rastgegenelement elastisch nachgiebig und das andere unnachgiebig ist.

Die Kupplungsfeder kann insbesondere ein elastischer Biegebalken sein, der entweder nur einseitig eingespannt ist und von seiner Einspannung frei vorragt, vorzugsweise in Umfangsrichtung um die Drehachse, falls die Rutschkupplung als Drehrutschkupplung gebildet ist. Besonders bevorzugt ist die Kupplungsfeder als beidseits eingespannter Biegebalken gebildet. Die betreffende Kupplungshälfte kann hierfür beispielsweise als Hülse mit innenliegender Drechachse geformt sein, zumindest in einem das Rastelement oder Rastgegenelement bildenden Abschnitt. Um den Biegebalken zu erhalten, ist die Hülse axial neben dem Biegebalken mit einem Durchbruch versehen, neben dem der Biegebalken als Brückenelement stehen bleibt. In Umfangsrichtung gesehen können auf diese Weise hintereinander mehrere Biegebalken mit dazwischen verbleibenden steiferen Hülsensegmenten erhalten werden.

In bevorzugten Ausführungen, in denen das Federglied bei der Bewegung des Betätigungsknopfs in die Dosierrichtung gespannt wird und die bei der Einstellung der Dosis aufgenommene Federenergie bei der Ausschüttung das Förderglied allein oder unterstützend antreibt, wirkt das Federglied vorteilhafterweise direkt oder gegebenenfalls auch erst über ein oder mehrere Zwischenglieder auf ein Dosierglied, das für die Einstellung der Dosis relativ zu dem Gehäuse hin und her beweglich ist. Bei der Ausschüttung wird das Dosierglied in eine der beiden Richtungen seiner Beweglichkeit bis gegen einen Ausschüttanschlag bewegt. Vorzugsweise ist es auch in die andere Richtung der Beweglichkeit bis gegen einen Maximaldosis-Anschlag beweglich. Die Beweglichkeit des Dosierglieds kann eine reine Translationsbeweglichkeit oder eine reine Rotationsbeweglichkeit sein, vorzugsweise handelt es sich um eine sowohl translatorische als auch rotatorische Beweglichkeit. So kann das Dosierglied insbesondere ein Gelenkelement eines Schraubgelenks sein. Im Falle eines drehbeweglichen Dosierglieds ist vorzugsweise wenigstens einer der genannten Anschläge ein Verdrehanschlag.

Obgleich es grundsätzlich genügt, wenn die Rutschkupplung nur ein einziges Rastelement und ein einziges Rastgegenelement oder in Ausführungen mit Übertragungsglied nur zwei Paare aus je nur einem Rastelement und einem Rastgegenelement umfasst, wird es bevorzugt, wenn pro Rasteingriff mehrere Rastelemente und mehrere Rastgegenelement vorgesehen sind. Es kann im jeweiligen Rasteingriff auch nur ein einziges Rastelement mit mehreren Rastgegenelementen verrasten, nämlich von Rastposition zu Rastposition jeweils mit dem nächsten Rastgegenelement. Hinsichtlich nur eines einzigen Rastgegenelements pro Rasteingriff gilt sinngemäß das gleiche. Bei mehreren Rastelementen oder Rastgegenelementen genügt es hinsichtlich des Merkmals der asymmetrischen Form, wenn nur eines der Elemente dieses Merkmal aufweist. Bevorzugt sind jedoch sämtliche Rastelemente oder Rastgegenelemente der Rutschkupplung so geformt.

Weitere bevorzugte Merkmale werden auch in den Unteransprüchen und deren Kombinationen beschrieben. Die dort beschriebenen Merkmale und die vorstehend erläuterten Ausgestaltungen ergänzen einander wechselseitig.

Nachfolgend wird ein Ausführungsbeispiel der Erfindung anhand von Figuren erläutert. An dem Ausführungsbeispiel offenbar werdende Merkmale bilden je einzeln und in jeder Merkmalskombination die Gegenstände der Ansprüche und die vorstehend beschriebenen Ausgestaltungen vorteilhaft weiter. Es zeigen:
- Figur 1: eine Injektionsvorrichtung in einer Ansicht,
- Figur 2: die Injektionsvorrichtung in einem Längsschnitt,
- Figur 3: den proximalen Abschnitt der Injektionsvorrichtung in dem Längsschnitt,
- Figur 4: eine Dosierkupplung in einem Kupplungseingriff,
- Figur 5: einen Gehäuseabschnitt und einen Betätigungsknopf und
- Figur 6: eine Ausschüttkupplung außer Eingriff.

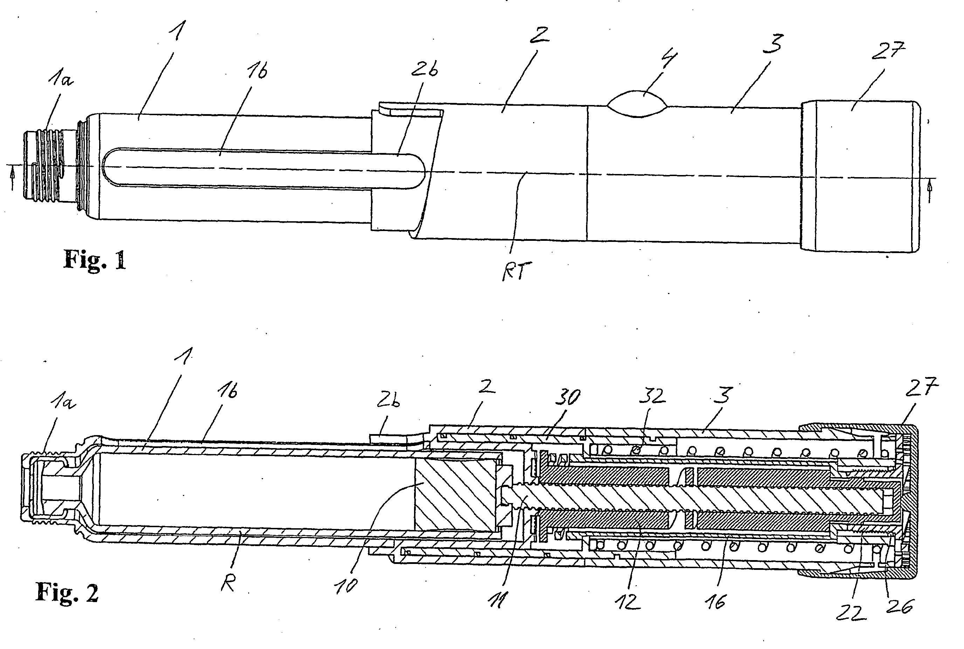

Figur 1 zeigt eine Injektionsvorrichtung in einer Seitenansicht. Die Injektionsvorrichtung ist als langgestreckter, schlanker Injektionspen gebildet. Sie umfasst ein Gehäuse mit einem distalen Gehäuseabschnitt 1 und einem proximalen Gehäuseabschnitt, der zwei fest miteinander verbundene Gehäuseabschnitte 2 und 3 umfasst. Die Injektionsvorrichtung dient der Verabreichung eines flüssigen Medikaments, beispielsweise Insulin. Das Medikament ist in einem Reservoir enthalten, das in dem Gehäuseabschnitt 1 austauschbar eingesetzt ist. Das Medikament ist durch einen Auslass 1a, der an dem distalen Ende des Gehäuseabschnitts 1 geformt ist, verabreichbar. Die Injektionsvorrichtung ist in Bezug auf eine zentrale Längsachse RT im Wesentlichen rotationssymmetrisch. Die Gehäuseabschnitte 1-3 sind je aus undurchsichtigem Kunststoffmaterial geformt. Um die Durchsicht auf das Reservoir zu ermöglichen, weist der Gehäuseabschnitt 1 ein Fenster 1b auf, das sich bis oder nahe bis zu dem proximalen Ende des Gehäuseabschnitts 1 und bis nahe zu dem Auslass 1a erstreckt, in Umfangsrichtung um die Achse RT jedoch vergleichsweise schmal ist. Der Gehäuseabschnitt 2 weist ebenfalls ein Fenster 2b auf, das im verbundenen Zustand der Gehäuseabschnitte 1 und 2 das Fenster 1b überdeckt, so dass auch im Verbindungsbereich der Gehäuseabschnitte 1 und 2 die Durchsicht auf das Reservoir frei ist. Die Fenster 1b und 2b sind in Umfangsrichtung gleich breit, unterschiedliche Abmessungen in Umfangsrichtung wären jedoch durchaus denkbar, solange zumindest die Funktion der Durchsicht auf das Reservoir erfüllt wird. In dem Gehäuseabschnitt 3 ist ein weiteres Fenster 4 in Form einer Lupe gebildet. Ein Betätigungsknopf 27 bildet das proximale Ende der Injektionsvorrichtung. Der Betätigungsknopf 27 erfüllt die Funktion eines manuell bedienbaren Dosierknopfs, durch dessen Betätigung eine zu verabreichende Dosis des Medikaments auswählbar bzw. einstellbar ist, und ferner die Funktion eines Auslöseknopfs, durch dessen Betätigung die eingestellte Dosis ausschüttbar ist. Die eingestellte Dosis ist durch das Fenster 4 auf einer unter dem Fenster 4 bei dem Einstellen durchlaufenden Dosisskala ablesbar.

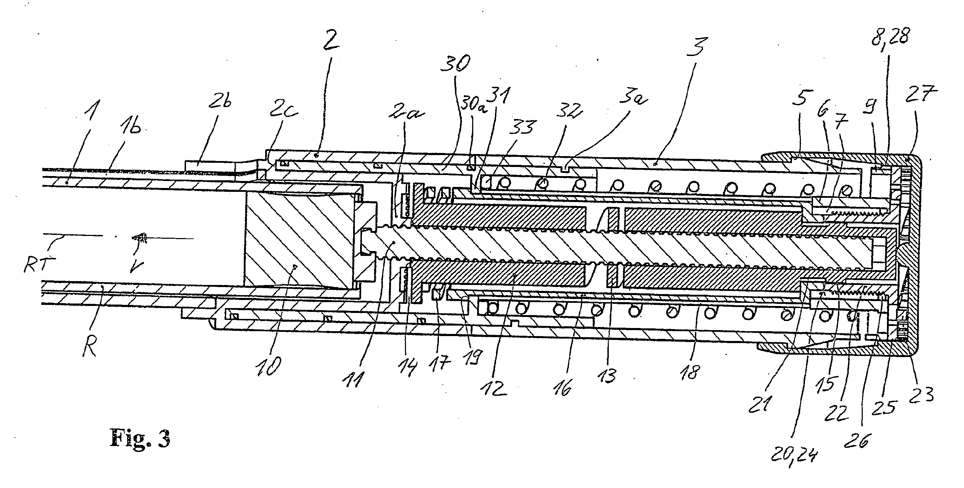

Die Figuren 2 und 3 zeigen die Injektionsvorrichtung je in einem die Längsachse RT enthaltenden Längsschnitt, die Figur 2 im Ganzen und die Figur 3 den proximalen Abschnitt in einer vergrößerten Darstellung.

Das Reservoir R ist eine Ampulle aus Glas oder durchsichtigem Kunststoff. In dem Reservoir R ist ein Kolben 10 in eine Vortriebsrichtung V auf den Auslass 1a zu axial beweglich aufgenommen. In dem in den Figuren 2 und 3 dargestellten Ausgangszustand ist das Reservoir R vollständig gefüllt, und der das Reservoir R proximal verschließende Kolben 10 nimmt seine proximalste Position ein. Der Gehäuseabschnitt 1 bildet im Grunde lediglich einen Reservoirhalter. Er ist mit dem Gehäuseabschnitt 2 fest, aber für einen Austausch des Reservoirs 1 lösbar verbunden, beispielsweise verschraubt.

Die den proximalen Gehäuseabschnitt bildenden Abschnitte 2 und 3 sind ebenfalls fest miteinander verbunden, vorzugsweise stoffschlüssig, und können funktional als ein einziger Gehäuseabschnitt 2, 3 betrachtet werden. Der Gehäuseabschnitt 2, 3 lagert eine Dosier- und Vortriebseinrichtung, mittels der eine pro Injektion ausschüttbare Dosis des Medikaments einstellbar und die eingestellte Dosis durch Vortrieb des Kolbens 10 ausschüttbar ist. Der Betätigungsknopf 27 ist für die Einstellung der Dosis mittels einer Dosierkupplung mit der Dosier- und Vortriebseinrichtung gekoppelt.

Die Dosier- und Vortriebseinrichtung umfasst mehrere Glieder, die mittels der Dosierkupplung und einer Ausschüttkupplung bei der Einstellung der Dosis und deren Aufschüttung auf unterschiedliche Weise miteinander gekoppelt sind. Eine Kolbenstange 11 bildet eines dieser Glieder. Bei der Ausschüttung drückt die Kolbenstange 11 gegen die Rückseite des Kolbens 10, so dass dieser sich in die Vortriebsrichtung V bewegt und Medikament durch den Auslass 1a ausgeschüttet wird. Die Kolbenstange 11 ist mit dem Gehäuseabschnitt 2 in einem Gewindeeingriff, für den sie über den größten Teil ihrer Länge mit einem Gewinde versehen ist. Der Gehäuseabschnitt 2 bildet das Gegengewinde an einer nach radial einwärts gegen die Kolbenstange 11 ragenden Halteeinrichtung 2a (Figur 3). Die Vortriebsbewegung der Kolbenstange 11 ist eine Drehbewegung um die Längsachse RT mit überlagerter Translation in die Vortriebsrichtung V. Das Gewinde der Kolbenstange 11 ist nicht umlaufend gebildet, sondern wird zumindest durch eine axiale Flachseite oder Nut unterbrochen. Dem Gewindeeingriff mit der Halteeinrichtung 2a und der im Gewindeeingriff gegebenen Halterung der Kolbenstange 11 tut dies jedoch keinen Abbruch.

Die Dosier- und Vortriebseinrichtung umfasst ferner ein erstes Kupplungsglied 12, ein zweites Kupplungsglied 16 und ein drittes Kupplungsglied 22. Das Kupplungsglied 12 ist mit der Kolbenstange 11 längs deren Flachseite oder Nut in einem Führungseingriff axial beweglich, aber verdrehgesichert verbunden. Das Kupplungsglied 12 ist proximal von der Halteeinrichtung 2a angeordnet und drückt in die Vortriebsrichtung V gegen die Halteeinrichtung 2a. Das Kupplungsglied 12 umgibt die Kolbenstange 11. Das Kupplungsglied 12 umfasst einen distalen Abschnitt, der die Halteeinrichtung 2a kontaktiert, und einen proximalen Abschnitt, der sich bis zu dem Betätigungsknopf 27 erstreckt. Zwischen den beiden Abschnitten ist eine Feder 13 gebildet. An dem distalen Ende des Kupplungsglieds 12 ist eine weitere Feder 14 geformt, die mehrere in Richtung auf die Halteeinrichtung 2a ragende Federzungen umfasst, die elastisch gespannt sind. Die Federzungen der Feder 14 bilden Rastelemente, die mit Rastgegenelementen der Halteeinrichtung 2a verrasten, so dass zwischen dem Kupplungsglied 12 und der Halteeinrichtung 2a und somit dem Gehäuseabschnitt 2 eine Drehrutschkupplung erhalten wird, die verhindert, dass die Kolbenstange 11 sich bei Erschütterungen relativ zu dem Gehäuse 1-3 drehen kann. Die im Kupplungseingriff der Rastelemente und Rastgegenelemente der Drehrutschkupplung übertragbare Kraft ist jedoch nicht so groß, dass sie die für das Ausschütten der eingestellten Dosis erforderliche Drehbewegung der Kolbenstange 11 verhindert oder in einem praktisch relevanten Ausmaß auch nur behindert. In dem Abschnitt der Feder 13 kann das Kupplungsglied 12 axial einfedern. Das Kupplungsglied 12 ist einschließlich der beiden Federn 13 und 14 in einem Stück aus Kunststoff geformt, vorzugsweise im Spritzguss.

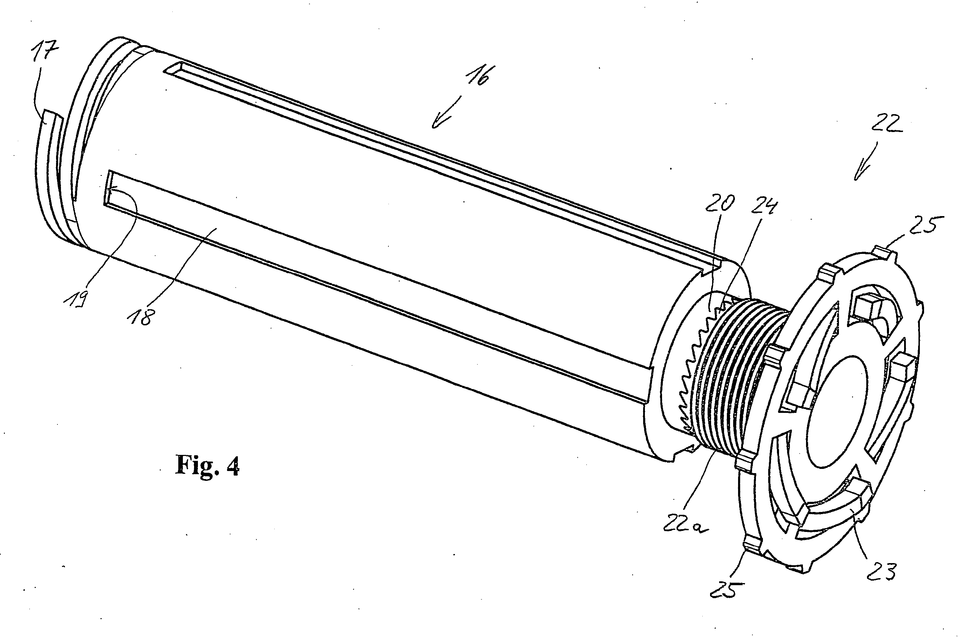

Das Kupplungsglied 16 ist um die Achse RT drehbar gelagert. Es ist hülsenförmig und umgibt das Kupplungsglied 12. An dem distalen Ende des Kupplungsglieds 16 ist eine Feder 17, im Ausführungsbeispiel eine axial kurze Schraubenfeder, in einem Stück angeformt. Das Kupplungsglied 16 stützt sich mit seiner Feder 17 in die distale Richtung an dem Kupplungsglied 12 ab und drückt dieses gegen die Halteeinrichtung 2a. An dem proximalen Ende des Kupplungsglieds 16 sind Eingriffselemente 20 geformt, die in dem dargestellten Zustand der Injektionsvorrichtung in einem lösbaren Kupplungseingriff mit Eingriffsgegenelementen 24 des Kupplungsglieds 22 sind. Die Eingriffselemente 20 und Eingriffsgegenelemente 24 können axial vom Kupplungsglied 16 oder 22 in Richtung auf das jeweils andere ragende Zähne sein, die zwei im Kupplungseingriff ineinander greifende, zu der Achse RT konzentrische Zahnkränze mit vorzugsweise gleichmäßiger Zahnteilung bilden, wie dies in Figur 4 beispielhaft dargestellt ist. Im dargestellten Zustand, in dem der Benutzer die zu verabreichende Dosis einstellen kann, greifen die Eingriffselemente 20 und Eingriffsgegenelemente 24 der beiden Kupplungsglieder 16 und 22 ineinander. Die Kupplungsglieder 16 und 22 bilden die Dosierkupplung und sind in dem Kupplungseingriff verdrehgesichert miteinander verbunden.

Figur 4 zeigt die Kupplungshälften der Dosierkupplung, nämlich die Kupplungsglieder 16 und 22, im Kupplungseingriff und herausgelöst aus dem Gesamtzusammenhang, so dass deren Funktionselemente 17-20 einerseits und 23-25 andererseits deutlich erkennbar sind.

Das Kupplungsglied 22 ist ebenfalls hülsenförmig und weist an seinem proximalen Ende einen nach radial außen ragenden Flansch auf. An dem äußeren Umfang des Flansches sind Eingriffselemente 25 geformt, die das Kupplungsglied 22 verdrehgesichert mit dem Betätigungsknopf 27 verbinden. Das Kupplungsglied 22 stützt sich über eine Feder 23 axial an dem Betätigungsknopf 27 ab. Die Feder 23 ist integrierter Bestandteil des Kupplungsglieds 22, indem das Kupplungsglied 22 in einem Stück einschließlich der Feder 23 aus Kunststoff geformt ist. Die Feder 23 besteht ähnlich wie die Feder 14 aus mehreren, um die Achse RT gebogenen sowie axial abragenden Federzungen. Sie sorgt in Verbindung mit der Feder 17 dafür, dass die Kupplungsglieder 16 und 22 axial elastisch in dem Kupplungseingriff gehalten werden. Die Feder 17 drückt das Kupplungsglied 16 und die Feder 23 drückt das Kupplungsglied 22 des Weiteren je axial auf Anschlag gegen den Gehäuseabschnitt 3.

Das Kupplungsglied 22 ist mit einem Stoppglied 26 in einem Gewindeeingriff. Hierfür ist das Kupplungsglied 22 an seinem äußeren Umfang vor dem Flansch mit einem Gewinde 22a versehen (Fig. 4). Der Gehäuseabschnitt 3 bildet dem Gewinde 22a radial außen gegenüberliegend eine Axialführung 7 für das Stoppglied 26, so dass das Stoppglied 26 bei einer Drehbewegung des Kupplungsglieds 22 im Gewindeeingriff axial bewegt wird. Der axiale Verstellweg des Stoppglieds 26 entspricht der Menge des Medikaments, die maximal, d. h. bei vollem Reservoir R, zur Verfügung steht und in mehreren Injektionen verabreicht wird. Bei einer Drehbewegung des Kupplungsglieds 22 wandert das Stoppglied 26 geführt von der Axialführung 7 längs des Außengewindes 22a des Kupplungsglieds 22 in die axiale Richtung, im Ausführungsbeispiel in die Vortriebsrichtung V. Wenn das Stoppglied 26 in dieser Bewegungsrichtung an einem durch eine innere Hülse 6 gebildeten Axialanschlag angelangt ist, bedeutet dies, dass das Reservoir R vollständig entleert wurde. Die innere Hülse 6 bildet die Axialführung 7 und ist relativ zu dem Gehäuseabschnitt 3 axial nicht beweglich, im Ausführungsbeispiel ist sie über einen radialen Verbindungssteg an dem Gehäuseabschnitt 3 geformt, d. h. sie ist Bestandteil des Gehäuseabschnitts 3.

Die Dosier- und Vortriebseinrichtung umfasst ferner ein Dosier- und Anzeigeglied 30, das mit dem Gehäuseabschnitt 3 in einem Gewindeeingriff ist. Für diesen Gewindeeingriff ist der Gehäuseabschnitt 3 mit einem Innengewinde 3a versehen. Der Gehäuseabschnitt 2 ist im Kontaktbereich mit dem Dosier- und Anzeigeglied 30 glatt. Das Gewinde 3a ist unmittelbar an der Mantelinnenfläche der kreiszylindrischen Hüllschale des Gehäuseabschnitts 2, 3 gebildet. Das Dosier- und Anzeigeglied 30 ist im Wesentlichen eine einfach kreiszylindrische Hülse mit einem entsprechend geformten Außengewinde 30a. Die Gewinde 3a und 30a haben eine deutlich größere Gewindesteigung als die Gewinde der Kolbenstange 11 und der Halteeinrichtung 2a. Die Gewindesteigung ist so groß, dass eine Selbsthemmung im Gewindeeingriff verhindert wird und das Dosier- und Anzeigeglied 30 unter einer reinen Axialkraft im Gewindeeingriff relativ zu dem Gehäuseabschnitt 2, 3 dreht und axial bewegt wird.

Das Dosier- und Anzeigeglied 30 ist axial beweglich, aber verdrehgesichert mit dem Kupplungsglied 16 gekoppelt. Im Ausführungsbeispiel sind das Dosier- und Anzeigeglied 30 und das Kupplungsglied 16 unmittelbar miteinander in einem entsprechenden Führungseingriff. Das Kupplungsglied 16 bildet hierfür eine axiale Führung 18 an seinem äußeren Umfang. An der Mantelinnenfläche des Dosier- und Anzeigeglieds 30 ist ein Eingriffselement 31 abragend geformt, mit dem das Dosier- und Anzeigeglied 30 in Führungseingriff mit dem Kupplungsglied 16, d. h. mit dessen Führung 18, ist. Die Führung 18 erstreckt sich über den größten Teil der Länge des Kupplungsglieds 16 und wird von dem Dosier- und Anzeigeglied 30 bei Einstellung der Maximaldosis über den größten Teil ihrer Länge durchfahren. Die Führung 18 wird von axialen Nuten in der Mantelaußenfläche des Kupplungsglieds 16 gebildet (Fig. 4). An ihren distalen Enden bilden die Nuten je einen Anschlag 19 für das Eingriffselement 31.

Die Dosier- und Vortriebseinrichtung umfasst schließlich ein Federglied 32, welches das Dosier- und Anzeigeglied 30 in die distale Richtung mit einer Kraft beaufschlagt. Das Federglied 32 des Ausführungsbeispiels ist eine Schraubenfeder und wirkt als Druckfeder. Das Federglied 32 stützt sich in die proximale Richtung an dem Gehäuseabschnitt 3 ab. Das Federglied 32 stützt sich in die distale Richtung an einer ringförmigen Gleitscheibe 33 ab, die zwischen das Kupplungsglied 16 und das Dosier- und Anzeigeglied 30 eingelegt ist und sich ihrerseits an dem Eingriffselement 31 abstützt. Mittels der Gleitscheibe 33 wird das Federglied 32 weitestgehend von Drehbewegungen des Dosier- und Anzeigeglieds 30 entkoppelt.

Das Dosier- und Anzeigeglied 30 ist relativ zu dem Kolben 10 und der Kolbenstange 11 sowie im Übrigen zu den Kupplungsgliedern 12, 16 und 22 zwischen axialen Endpositionen hin und her beweglich. Die beiden Endpositionen sind eine Nulldosis-Position und eine Maximaldosis-Position. Die beiden Endpositionen werden je durch einen Anschlag vorgegeben. Den Anschlag für die Nulldosis-Position bildet eine Schulter 2c an dem distalen Ende des Gehäuseabschnitts 2 als Axialanschlag. Den Anschlag für die Maximaldosis-Position bildet der Gehäuseabschnitt 3. In der Nulldosis-Position überlappt ein distaler Abschnitt des Dosier- und Anzeigeglieds 30 das Reservoir R in die Vortriebsrichtung V bis über den Kolben 10 hinaus, wenn der Kolben 10 wie in den Figuren 2 und 3 dargestellt eine in dem Reservoir R hinterste Position einnimmt. Der Kolben 10 nimmt diese Position dann ein, wenn das Reservoir R vollständig gefüllt ist. Das Gewinde 30a erstreckt sich bis an das distale Ende oder zumindest bis nahe an das distale Ende des Dosier- und Anzeigeglieds 30, so dass auch das Gewinde 30a das Reservoir R und den Kolben 10 axial überlappt, wenn das Dosier- und Anzeigeglied 30 die Nulldosis-Position einnimmt. Das Gewinde 30a endet vor dem distalen Ende des Dosierglieds 30 und bildet zusammen mit dem Gewinde 3a des Gehäuseabschnitts 3 einen Verdrehanschlag, der die Maximaldosis-Position des Dosier- und Anzeigeglieds bestimmt. Das Gewinde 30a könnte alternativ auch an dem distalen Ende des Dosier- und Anzeigeglieds 30 auslaufen; der die Maximaldosis-Position bestimmende Anschlag wäre in solch einer Modifikation anderweitig zu bilden.

Das Dosier- und Anzeigeglied 30 ist an seinem distalen Ende nicht umlaufend vollzylindrisch, sondern nur teilzylindrisch, um trotz der axialen Überlappung den Blick auf den Kolben auch dann freizugeben, wenn das Dosier- und Anzeigeglied 30 wie bevorzugt nicht aus einem durchsichtigen Kunststoffmaterial, sondern aus einem undurchsichtigen oder allenfalls opaken Kunststoffmaterial oder einem anderen Material geformt ist. Der distale Rand des Dosier- und Anzeigeglieds 30 verläuft spiralförmig um die Achse RT, so dass das Dosier- und Anzeigeglied 30 wie in den Figuren 2 und 3 erkennbar mit seinem distalen Ende in einem Umfangsbereich axial sogar über den Kolben 10 hinaussteht und in einem anderen Umfangsbereich, der in der Nulldosis-Position mit dem Fenster in Überdeckung ist, hinter dem Kolben 10 zurücksteht, so dass der Kolben 10 durch die Fenster 1b und 2b beobachtet werden kann. Anstatt eines spiralförmigen Rands kann das Dosier- und Anzeigeglied 30 umlaufend mit Ausnahme beispielsweise einer Durchbrechung überall bis zu der gleichen axialen Höhe reichen und so mit Ausnahme des durchbrochenen Bereichs überall über den Kolben 10 in die Vortriebsrichtung V vorstehen, wobei die Durchbrechung in der Nulldosis-Position in Überdeckung mit dem Fenster 2b wäre. Die Schulter 2c des Gehäuseabschnitts 2 ist dem spiraligen Verlauf des distalen Rands des Dosier- und Anzeigeglieds 30 folgend ebenfalls um die Achse RT gewunden. Der gewundene Verlauf der Schulter 2c lässt sich auch aus Figur 1 ersehen. Der Gehäuseabschnitt 2 umfasst eine äußere Hülse, die zusammen mit dem Gehäuseabschnitt 3 die Dosier- und Vortriebseinrichtung umhüllt, und eine innere Hülse, die an ihrem distalen Ende durch einen die Schulter 2c bildenden Steg mit der äußeren Hülse verbunden ist und an deren proximalem Ende die Halteeinrichtung 2a nach radial einwärts vorragt. Zwischen der äußeren und der inneren Hülse verbleibt ein Ringspalt, in den das Dosier- und Anzeigeglied 30 über einen größeren Teil seiner Länge in der Nulldosis-Position hineinragt, wobei der größere Teil im Bereich des längsten Unfangssegments des Dosier- und Anzeigeglieds 30 etwa 50 % der gesamten Länge des Dosier- und Anzeigeglieds 30 ausmacht.

Das Dosier- und Anzeigeglied 30 ist Träger einer Dosisskala, die an dem äußeren Umfang des Dosier- und Anzeigeglieds 30 spiralig umläuft mit einer in Bezug auf die Achse RT gemessenen Steigung, die der Steigung des Gewindes 30a entspricht. Die Dosisskala besteht aus Markierungen und Ziffern, wobei jede der Markierungen einer kleinsten einstellbaren Dosiseinheit entspricht. Die Dosisskala ist während der Einstellung der zu verabreichenden Dosis durch das Fenster 4 des Gehäuseabschnitts 3 ablesbar (Figur 1).

Der Betätigungsknopf 27 bildet das proximale Ende der Injektionsvorrichtung. Er ist mit dem Gehäuseabschnitt 3 verclippt, indem der Gehäuseabschnitt 3 an seinem proximalen Ende mit einer Schulter 5 versehen ist, die der Betätigungsknopf 27 hintergreift. Der Betätigungsknopf 27 ist relativ zu dem Gehäuse 1-3 um die Achse RT in eine Dosierrichtung und eine Korrekturrichtung drehbar. Bei dem Einstellen der Dosis wird durch die Drehung des Betätigungsknopfs 27 in die Dosierrichtung die Dosis vergrößert und bei einer Drehbewegung in die Korrekturrichtung verringert, wodurch eine versehentlich zu groß eingestellte Dosis korrigiert werden kann. Der Betätigungsknopf 27 ist ferner mit dem Kupplungsglied 22 axial beweglich, aber mittels der Eingriffselemente 25 verdrehgesichert verbunden.

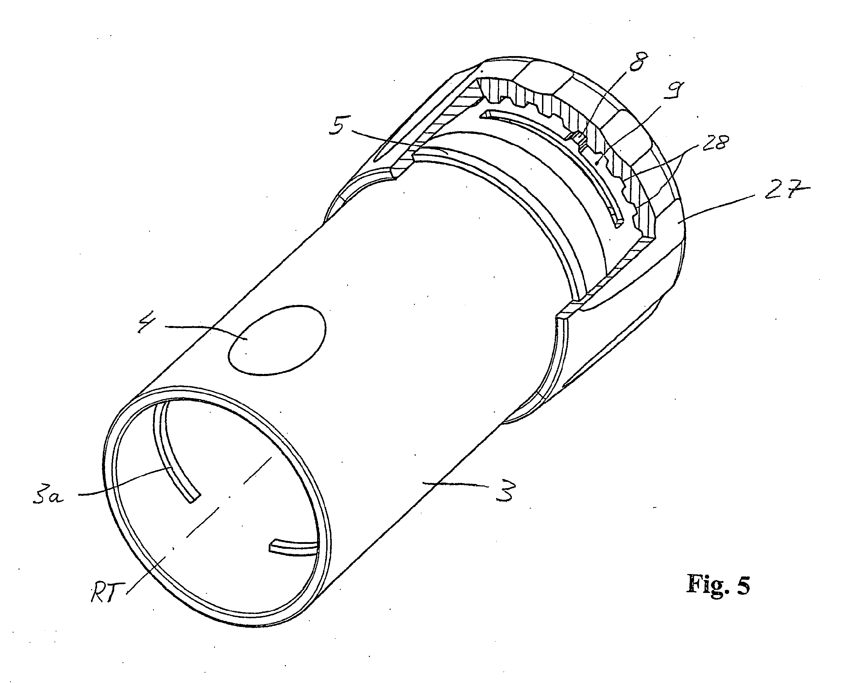

Die Figur 5 zeigt den Gehäuseabschnitt 3 und den Betätigungsknopf 27 im Kupplungseingriff, wobei ein Teil des Betätigungsknopfs für die Darstellung des Eingriffs weggeschnitten ist.

Der Betätigungsknopf 27 bildet über die Verdrehsicherung mit dem Kupplungsglied 22 hinaus eine Rutschkupplung, im Ausführungsbeispiel eine Drehrutschkupplung, mit dem Gehäuseabschnitt 3. An dem Betätigungsknopf 27 sind für die Bildung der Rutschkupplung um die Achse RT gleichmäßig verteilt mehrere Rastelemente 28 geformt, die mit korrespondierenden an dem distalen Ende des Gehäuseabschnitts 3 geformten, vorzugsweise kongruenten Rastgegenelementen 8 in einem Kupplungseingriff sind. Die Anzahl der Rastgegenelemente 8 ist geringer als die Anzahl der Rastelemente 28. Der Betätigungsknopf 27 ist bei bestehendem Kupplungseingriff der Rastelemente 28 und Rastgegenelemente 8 relativ zu dem Gehäuseabschnitt 3 um die Achse RT drehbar, wobei die Rastelemente 28 und Rastgegenelemente 8 in diskret über den Umfang verteilten Rastpositionen paarweise lösbar miteinander verrasten. Die Teilung der Rastelemente 28 und somit der im Winkel- oder Bogenmaß zwischen je in Umfangsrichtung nächstbenachbarten Rastelementen 28 gemessene Abstand korrespondiert mit der kleinsten einstellbaren Dosiseinheit, die Teilung der Rastgegenelemente einem ganzzahligen Vielfachen derselben Dosiseinheit. Die Rutschkupplung muss nicht unbedingt zwischen dem Gehäuse 1-3 und dem Betätigungsknopf 27 unmittelbar gebildet sein. Anstatt die Rastelemente 28 an dem Betätigungsknopf 27 vorzusehen, könnten sie auch an dem Kupplungsglied 22 geformt sein. Modifizierte Rastelemente und Rastgegenelemente könnten in beiden Ausführungen beispielsweise axial anstatt radial ineinander greifen.

Die Rastelemente 28 und entsprechend die Rastgegenelemente 8 sind bezüglich der Drehrichtung asymmetrisch geformt, so dass die für das Lösen des Rasteingriffs in eine Kupplungsdosierrichtung aufzuwendende Kraft größer ist als in die Gegendrehrichtung, die Kupplungskorrekturrichtung. Da im Ausführungsbeispiel der Betätigungsknopf 27 die Rastelemente 28 unmittelbar selbst bildet, ist die Dosierrichtung des Betätigungsknopfs 27 gleichzeitig auch die Kupplungsdosierrichtung, und die Korrekturrichtung ist die Kupplungskorrekturrichtung. Die Rastgegenelemente 8 sind Rastnocken, die von der Mantelaußenfläche des Gehäuseabschnitts 3 nach radial auswärts vorragen. Die Rastelemente 28 sind entsprechend an der zugewandt gegenüberliegenden Mantelinnenfläche des Betätigungsknopfs 27 als korrespondierende Vertiefungen oder Zahnlücken einer Innenverzahnung gebildet. Um die Asymmetrie bezüglich der beiden Drehrichtungen zu erzielen, sind die in Kupplungsdosierrichtung weisenden, vorlaufenden Flanken der Rastelemente 28 flacher als die in die Gegendrehrichtung weisenden, bei Bewegung der Rastelemente 28 in die Kupplungsdosierrichtung nachlaufenden Flanken. Die Rastgegenelemente 8 sind korrespondierend mit beiden Flanken angeschmiegt geformt.

Die Asymmetrie der Rutschkupplung in Bezug auf die beiden Drehrichtungen des Betätigungsknopfs 27 ist auf die Richtung der von dem Kraftglied 32 auf das Dosier- und Anzeigeglied 30 ausgeübten Kraft abgestimmt. Bei einer Drehbewegung des Betätigungsknopfs 27 in Richtung einer Vergrößerung der Dosis, dem Aufdosieren, wird nämlich über die bei 20, 24 gebildete Dosierkupplung und den bei 18 und 31 gebildeten Führungseingriff das Dosier- und Anzeigeglied 30 im Gewindeeingriff verdreht und in die proximale Richtung bewegt. Im Verlaufe der Translation in die proximale Richtung wird das Federglied 32 zunehmend gespannt. Die Elastizitätskraft des Federglieds 32 setzt der Bewegung des Dosier- und Anzeigeglieds 30 in die proximale Richtung einen Widerstand entgegen, der über die beschriebene Kopplung auf die Rutschkupplung 8, 28 wirkt und der Drehbewegung des Betätigungsknopfs 27 in die Dosierrichtung, in welche die flacheren Flanken der Rastelemente 28 und Rastgegenelemente 8 weisen, einen zusätzlichen, beim Aufdosieren zunehmenden Reibwiderstand entgegensetzt. Die von dem Federglied 32 ausgeübte Kraft unterstützt umgekehrt die Drehbewegung in Richtung einer Dosiskorrektur.

Figur 5 zeigt den Gehäuseabschnitt 3 und insbesondere auch dessen Rastgegenelemente 8 für die Rutschkupplung 3, 27. Der Gehäuseabschnitt 3 bildet in einem proximalen Hülsenabschnitt radial elastisch nachgiebige Kupplungsfedern 9, eine Kupplungsfeder 9 pro Rastgegenelement 8. Die Rastgegenelemente 8 ragen von den Kupplungsfedern 9 jeweils radial nach außen vor. Die Kupplungsfedern 9 sind Ringsegmente. Um die Achse RT umlaufend setzt sich der Hülsenabschnitt aus alternierend den Kupplungsfedern 9 und demgegenüber steiferen Ringsegmenten zusammen. Die Kupplungsfedern 9 wirken je als in Umfangsrichtung erstreckte Biegebalken, die beidseits eingespannt sind, nämlich an den beiden je nächstbenachbarten steiferen Ringsegmenten. In Umfangsrichtung erstreckt sich längs der Kupplungsfedern 9 je eine Ausnehmung, wodurch die Nachgiebigkeit der jeweiligen Kupplungsfeder 9 erhöht wird. Der die Kupplungsfedern 9 bildende Hülsenabschnitt bildet das proximale Ende des Gehäuseabschnitts 3.

Der Betätigungsknopf 27 ist topfförmig mit einem das distale Ende der Injektionsvorrichtung bildenden Boden und einer von dem Boden aufragenden, um die Achse RT umlaufenden Wand, an deren Innenfläche die Rastelemente 8 über den Umfang gleichmäßig verteilt angeordnet und je als axial erstreckte Vertiefung geformt sind. Die Rastelemente 28 sind im montierten Zustand sowohl mit den Rastgegenelementen 8 als auch mit den Eingriffselementen 25 (Fig. 3) des Kupplungsglieds 22 im Eingriff, wobei sowohl der Rasteingriff mit den Rastgegenelementen 8 als auch der Verdrehsicherungseingriff mit den Eingriffselementen 25 in jeder Axialposition des Betätigungsknopfs 27 erhalten bleibt.

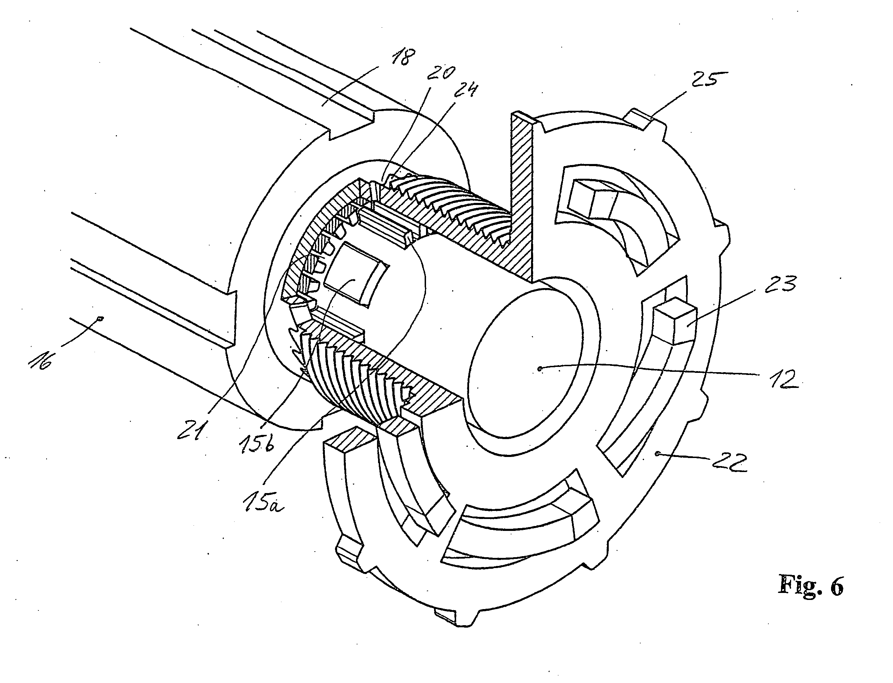

Die Dosier- und Vortriebseinrichtung enthält über die von den Kupplungsgliedern 16 und 22 gebildete Dosierkupplung hinaus eine zweite Kupplung, nämlich die bereits ebenfalls genannte Ausschüttkupplung, die in Figur 6 gezeigt ist. Die Ausschüttkupplung wird von den Kupplungsgliedern 12 und 16 gebildet, die hierfür mit im Kupplungseingriff ineinander greifenden Eingriffselementen versehen sind. Auf der Seite des Kupplungsglieds 12 handelt es sich um Eingriffselemente 15a und 15b. Das Kupplungsglied 16 ist mit Eingriffsgegenelementen 21 versehen. Die Eingriffselemente 15a sind axiale Rippen, die an dem proximalen Ende von einer Mantelaußenfläche des Kupplungsglieds 12 nach radial außen abragen. Die Eingriffsgegenelemente 21 sind an einer Mantelinnenfläche an dem proximalen Ende des Kupplungsglieds 16 korrespondierend als axiale Sacknuten geformt. Die Eingriffsgegenelemente 21 bilden eine axiale Führung für die Eingriffselemente 15a. Die Eingriffselemente 15a bilden eine Art Außenverzahnung um die Achse RT. Die Außenverzahnung ist an wenigstens einer Stelle unterbrochen, im Ausführungsbeispiel an zwei einander diametral gegenüberliegenden Stellen. In dem unterbrochenen Umfangsbereich ist je eines der Eingriffselemente 15b geformt. Die Eingriffselemente 15b ragen wie die Eingriffselemente 15a nach radial außen vor, sind jedoch in Umfangsrichtung breiter als die Eingriffselemente 15a, so dass sie nicht in die als Sacknuten gebildeten Eingriffselemente 21 einfahren können. Vielmehr bilden die zwischen den Eingriffselementen 21 verbleibenden Rippenstege einen Axialanschlag für die Eingriffselemente 15b, wenn das Kupplungsglied 12 relativ zu dem Kupplungsglied 16 in die distale Richtung bewegt wird.

Das Kupplungsglied 16 ist wahlweise mit dem Kupplungsglied 22 oder dem Kupplungsglied 12 in einem Kupplungseingriff, d. h. bei gelöster Ausschüttkupplung im Kupplungseingriff mit dem Kupplungsglied 22 und bei gelöster Dosierkupplung im Kupplungseingriff mit dem Kupplungsglied 12. Für die Einstellung der Dosis ist die Dosierkupplung geschlossen, d. h. die Eingriffselemente 15a und 15b sind in axialer Überdeckung nur mit dem Kupplungsglied 22, dessen Mantelinnenfläche jedoch umlaufend glatt ist, so dass bei der Einstellung der Dosis das Kupplungsglied 22 relativ zu dem Kupplungsglied 12 drehen kann. Für die Ausschüttung der eingestellten Dosis wird das Kupplungslied 12 in den Kupplungseingriff mit dem Dosier- und Anzeigeglied 16 bewegt, die Dosierkupplung wird dadurch gelöst und die Ausschüttungskupplung geschlossen, so dass die Ausschüttung beginnen kann. Fig. 6 zeigt die Kupplungsglieder 12, 16 und 22 im Zustand der geschlossenen Dosierkupplung 16, 22.

### Im Folgenden wird der Gebrauch der Vorrichtung erläutert:

Der Benutzer hält die Injektionsvorrichtung in einer Hand und setzt mit der anderen Hand eine Kanüleneinheit auf den Auslass 1a auf und schraubt sie fest. Ansonsten befindet sich die Injektionsvorrichtung in dem in den Figuren 1 bis 3 gezeigten Zustand. Durch die Fenster 1b und 2b kann der Benutzer den Füllstand des Reservoirs R bzw. die Position des Kolbens 10 verifzieren. Es sei angenommen, dass er das Reservoir R bereits entlüftet hat und sich im nächsten Schritt eine bestimmte Dosis des Medikaments injizieren möchte. Durch Drehen des Betätigungsknopfs 27 stellt er die gewünschte Dosis ein. Während des Einstellens kann er die der axialen Position des Dosier- und Anzeigeglieds 30 entsprechende momentane Dosis durch das Fenster 4 ablesen. Sollte er bei dem Vorgang des Aufdosierens versehentlich eine zu hohe Dosis gewählt haben, kann er die Überdosis durch Drehen des Betätigungsknopfs 27 in die Korrekturrichtung korrigieren. Während der Einstellung der Dosis wird die Drehbewegung des Betätigungsknopfs 27 über die bei 25 bestehende Verdrehsicherung auf das Kupplungsglied 22, über die bei 20, 24 geschlossene Dosierkupplung auf das Kupplungsglied 16 und im Führungseingriff zwischen 18 und 31 auf das Dosier- und Anzeigeglied 30 übertragen. Das Dosier- und Anzeigeglied 30 bewegt sich im Eingriff der Gewinde 3a und 30a rotatorisch um die Achse RT und translatorisch in die proximale Richtung. Das Federglied 32 wird bei dem Aufdosieren elastisch zunehmend gespannt und unterstützt eine gegebenenfalls vorzunehmende Dosiskorrektur. Sowohl beim Aufdosieren als auch bei einer Dosiskorrektur erzeugt der Rasteingriff der Rutschkupplung zwischen dem Gehäuseabschnitt 3 und dem Betätigungsknopf 27 ein deutlich vernehmbares Klickgeräusch. Aufgrund der Steilheit der Gewinde 3a und 30a legt das Dosier- und Anzeigeglied 30 einen entsprechend langen Verstellweg in axialer Richtung zurück. Entsprechend groß sind in axialer Richtung die Abstände zwischen den Dosismarken der Dosisskala des Dosier- und Anzeigeglieds 30, so dass eine eindeutige Ablesung der gerade unter dem Fenster 4 durchlaufenden Dosismarke auch für sehbehinderte Benutzer gewährleistet ist.

Die Kolbenstange 11 ist von der Dosierbewegung, im Ausführungsbeispiel Dosierdrehbewegung, entkoppelt und wird zusätzlich durch das umgebende, ebenfalls entkoppelte Kupplungsglied 12 gegen Drehbewegungen gesichert. Die Sicherung wird wie bereits erwähnt durch die zwischen dem Kupplungsglied 12 und der Halteeinrichtung 2a gebildete Rutschkupplung und den verdrehgesicherten Eingriff des Kupplungsglieds 12 mit der Kolbenstange 11 erhalten.

Wenn die gewünschte Dosis eingestellt ist, sticht der Benutzer die Einstechkanüle an der gewünschten Injektionsstelle in und unter die Haut in das subkutane Gewebe. Anschließend löst er mit der gleichen Hand, die auch die Injektionsvorrichtung bei dem Einstechen hält, die Ausschüttung der eingestellten Dosis aus.

Der Betätigungsknopf 27, der bei der Einstellung der Dosis die Funktion eines Dosierknopfs erfüllt, ist in Doppelfunktion auch Auslöseknopf. Während der Einstellung der Dosis wird das Dosier- und Anzeigeglied 30 entsprechend den Dosisrastpositionen der Rastelemente 28 und Rastgegenelemente 8 der Rutschkupplung gehalten, d. h. die Rutschkupplung verhindert, dass sich das Dosier- und Anzeigeglied 30 unter der Einwirkung des Federglieds 32 bewegen kann. Für die Ausschüttung muss daher die über die Rutschkupplung bei 8, 28 bestehende Kopplung zwischen dem Gehäuseabschnitt 3 und dem Dosier- und Anzeigeglied 30 gelöst werden. Dies geschieht durch Lösen der bei 20, 24 gebildeten Dosierkupplung, indem der Benutzer den Betätigungsknopf 27 mit dem Daumen in die distale Richtung presst. Bei Ausübung einer entsprechenden Druckkraft bewegt sich der Betätigungsknopf 27 relativ zu dem Gehäuseabschnitt 3 und dem Kupplungsglied 22 entgegen der Kraft der Feder 23 in die distale Richtung und drückt bei dieser Bewegung gegen das Kupplungsglied 12. Das Kupplungsglied 12 federt im Bereich seiner integrierten Feder 13 axial in sich ein, so dass seine Eingriffselemente 15a in den verdrehgesicherten Eingriff mit den Eingriffsgegenelementen 21 des Kupplungsglieds 16 gelangen. In einer Übergangsphase der Axialbewegung ist die Dosierkupplung zwischen dem Kupplungsglied 22 und dem Kupplungsglied 16 noch geschlossen, während auch bereits der Kupplungseingriff der Ausschüttkupplung zwischen dem Kupplungsglied 12 und dem Kupplungsglied 16 hergestellt ist. Sobald die Eingriffselemente 15b jedoch den Axialanschlag des Kupplungsglieds 12 in die distale Richtung kontaktieren, bewirkt anhaltender Druck auf den Betätigungsknopf 27, dass das Kupplungsglied 16 gegen die Kraft seiner Feder 17 in die distale Richtung bewegt und dadurch axial aus dem Kupplungseingriff mit dem Kupplungsglied 22 gehoben wird.

Sobald die Dosierkupplung gelöst ist, schraubt sich das Dosier- und Anzeigeglied 30 unter der Elastizitätskraft des Federglieds 32 in die distale Richtung. Der Drehbewegungsanteil des Dosier- und Anzeigeglieds 30 wird im Führungseingriff zwischen der Führung 18 und dem Eingriffsglied 31 auf das Kupplungsglied 16 übertragen. Das Kupplungsglied 16 überträgt wegen der geschlossenen Ausschüttkupplung die Drehbewegung auf das Kupplungsglied 12, das wiederum verdrehgesichert mit der Kolbenstange 11 verbunden ist, so dass sich die Kolbenstange 11 im Gewindeeingriff mit der Halteeinrichtung 2a dreht und den Kolben 10 in die Vortriebsrichtung V schiebt. Die Ausschüttbewegung der daran beteiligten Komponenten wird durch Anschlag des Dosier- und Anzeigeglieds 30 an der Schulter 2c beendet. Der Hub oder axiale Anteil des Verstellwegs des Dosier- und Anzeigeglieds 30 bestimmt daher den Hub des Kolbens 10 und somit die ausgeschüttete Dosis. Wegen der größeren Gewindesteigung der Gewinde 3a und 30a gegenüber den Gewinden der Kolbenstange 11 und der Halteeinrichtung 2a wird der Hub des Dosier- und Anzeigeglieds 30 in einen dem Untersetzungsverhältnis entsprechenden Hub des Kolbens 10 untersetzt. Während der Ausschüttbewegung erzeugt die zwischen der Feder 14 und der Halteeinrichtung 2a gebildete Rutschkupplung ein Klickgeräusch, das deutlich wahrnehmbar ist und dem Benutzer auch akustisch anzeigt, dass Medikament ausgeschüttet wird. Der Ausschüttvorgang ist ferner durch die Fenster 1b und 2b anhand der axialen Position des Kolbens 10 zumindest grundsätzlich verifizierbar.

Bei dem Einstellen der Dosis wird das Stoppglied 26 im Gewindeeingriff mit dem Kupplungsglied 22 entsprechend der eingestellten Dosis ebenfalls bewegt. Wenn die durch den maximalen Hub des Stoppglieds 26 vorgegebene Medikamentenmenge insgesamt verabreicht wurde, was bei vollständig gefülltem Reservoir R erst nach mehreren Injektionen der Fall ist, tauscht der Benutzer das entleerte Reservoir R gegen ein gefüllte neues Reservoir R aus. Er muss hierfür lediglich den Gehäuseabschnitt 1 von dem proximalen Gehäuseabschnitt 2, 3 trennen, das neue Reservoir R mit bereits darin aufgenommenem Kolben 10 einsetzen und die Gehäuseabschnitt 1 und 2, 3 wieder miteinander verbinden.

Im Ausführungsbeispiel sind mit Ausnahme des Kraftglieds 32 sämtliche Federn als integrierter Bestandteil jeweils eines Kupplungsglieds mit Kopplungsteil und Federteil gebildet. Eine oder mehrere der Federn kann alternativ jedoch auch in herkömmlicher Weise als Stahlfeder separat vom jeweiligen Kupplungsglied gebildet sein. So können die Federn 13, 17 und 23 ohne weiteres durch Stahlfedern ersetzt werden, da sie ausschließlich die Federfunktion erfüllen. Für den Ersatz der Feder 14 hingegen bedarf es einer Ersatzfeder und der Formung von Rastelementen am distalen Ende des Kupplungsglieds 12.

Zu der Anordnung aus Dosier- und Anzeigeglied 30 und Federglied 32 ist zu bemerken, dass anstatt des Dosier- und Anzeigeglieds 30 alternativ das Federglied 32 in axialer Überlappung mit dem Reservoir R angeordnet sein kann und sich an einem Widerlager, beispielsweise der Schulter 2c, abstützt. Bei einer derartigen Umkehrung der Einbauverhältnisse würde das Dosier- und Anzeigeglied 30 bei der Ausschüttung in die proximale Richtung bis gegen einen Anschlag bewegt werden. In noch einer weiteren Alternative kann das Federglied 32 als Zugfeder eingebaut sein, obgleich der Einbau als Druckfeder besonderes bevorzugt ist. Noch eine weitere Alternative wird in den deutschen Patentanmeldungen Nrn. 10 2005 043 806.7 und 10 2005 043 807.5 beschrieben. Gemäß diesen Anmeldungen ist das auf die Rutschkupplung wie beschrieben abgestimmte Federglied als Torsionsfeder eingebaut. Die älteren Anmeldungen werden hinsichtlich der abgestimmten Anordnung aus Rutschkupplung und Federglied einbezogen.

### Bezugszeichen:

- 1: Gehäuseabschnitt
- 1a: Auslass
- 1b: Fenster
- 2: Gehäuseabschnitt
- 2a: Halteeinrichtung
- 2b: Fenster
- 2c: Schulter
- 3: Gehäuseabschnitt
- 3a: Gewinde
- 4: Fenster
- 5: Schulter
- 6: Innere Hülse
- 7: Führung
- 8: Rastgegenelement
- 9: Kupplungsfeder
- 10: Kolben
- 11: Kolbenstange
- 12: Kupplungsglied
- 13: Feder
- 14: Feder
- 15a: Eingriffselement
- 15b: Eingriffselement
- 16: Kupplungsglied
- 17: Feder
- 18: Kopplungsteil, Führung
- 19: Anschlag
- 20: Eingriffselement
- 21: Eingriffsgegenelement, Führung
- 22: Kupplungsglied
- 22a: Gewinde
- 23: Feder
- 24: Eingriffsgegenelement
- 25: Eingriffselement
- 26: Stoppglied
- 27: Betätigungsknopf
- 28: Rastelement
- 29: -
- 30: Dosier- und Anzeigeglied
- 30a: Gewinde
- 31: Eingriffselement
- 32: Federglied
- 33: Gleitscheibe

- R: Reservoir
- RT: Längsachse
- V: Vortriebsrichtung

## Patentansprüche

1. Injektionsvorrichtung umfassend
a) ein Gehäuse (1-3) mit einem Reservoir (R) für ein injizierbares Produkt,
b) einen Betätigungsknopf (27), der für die Einstellung einer Produktdosis relativ zu dem Gehäuse (1-3) in eine die Dosis vergrößernde Dosierrichtung und eine Korrekturrichtung beweglich ist,
c) ein Förderglied (10) für die Ausschüttung der eingestellten Dosis,
d) eine Rutschkupplung (3, 27) mit einem Rastelement (28) und einem Rastgegenelement (8), die miteinander in einem Rasteingriff verrastend den Betätigungsknopf (27) bei einer Bewegung in die Dosierrichtung oder die Korrekturrichtung jeweils in diskreten Rastpositionen mit dem Gehäuse (1-3) form- und kraftschlüssig koppeln,
e) und ein Federglied (32), das der Bewegung des Betätigungsknopfs (27) in wenigstens eine der Richtungen eine Federkraft entgegensetzt,
f) **dadurch gekennzeichnet, daß** das Rastelement (28) oder das Rastgegenelement (8) so geformt sind, dass sie im Rasteingriff der Bewegung in die wenigstens eine der Richtungen einen geringeren Widerstand entgegen setzen als der Bewegung in die andere.

2. Injektionsvorrichtung nach Anspruch 1, wobei das Federglied (32) mit dem Förderglied (10) gekoppelt oder koppelbar ist und das Förderglied (32) bei der Ausschüttung antreibt.

3. Injektionsvorrichtung nach dem vorhergehenden Anspruch, wobei das Federglied (32) bei der Einstellung der Dosis von dem Förderglied (10) entkoppelt und für die Ausschüttung mittels einer Ausschüttkupplung (12, 16) mit dem Förderglied (10) koppelbar ist.

4. Injektionsvorrichtung nach einem der vorhergehenden Ansprüche, wobei das Federglied (32) bei der Bewegung des Betätigungsknopfs (27) in die wenigstens eine der Richtungen von Rastposition zu Rastposition stärker gespannt wird.

5. Injektionsvorrichtung nach einem der vorhergehenden Ansprüche, wobei das Federglied (32) der Bewegung in die Dosierrichtung die Federkraft entgegensetzt:

6. Injektionsvorrichtung nach einem der vorhergehenden Ansprüche, wobei das Federglied (32) eine Druckfeder oder Torsionsfeder ist.

7. Injektionsvorrichtung nach einem der vorhergehenden Ansprüche, wobei die Rutschkupplung eine erste Kupplungshälfte (27) mit dem Rastelement (28) und eine zweite Kupplungshälften (3) mit dem Rastgegenelement (8) umfasst und eine der Kupplungshälften (3, 27) relativ zu der anderen um eine Drehachse (RT) drehbeweglich ist.

8. Injektionsvorrichtung nach dem vorhergehenden Anspruch, wobei das Rastelement (28) und das Rastgegenelement (8) in dem Rasteingriff bezüglich der Drehachse (RT) radial ineinander greifen.

9. Injektionsvorrichtung nach Anspruch 7, wobei das Rastelement und das Rastgegenelement in dem Rasteingriff bezüglich der Drehachse (RT) axial ineinander greifen.

10. Injektionsvorrichtung nach einem der vorhergehenden Ansprüche, wobei die Rutschkupplung aus einer ersten Kupplungshälfte (27) mit dem Rastelement (28) und einer zweiten Kupplungshälfte (3) mit dem Rastgegenelement (8) besteht und das Rastelement (28) und das Rastgegenelement (8) unmittelbar miteinander in dem Rasteingriff sind.

11. Injektionsvorrichtung nach einem der vier vorhergehenden Ansprüche, wobei eine der Kupplungshälften (3, 27) relativ zu der anderen in die Dosierrichtung und die Korrekturrichtung beweglich ist.

12. Injektionsvorrichtung nach einem der vorhergehenden Ansprüche, wobei das Rastelement (28) oder das Rastgegenelement (8) bezüglich der Dosierrichtung asymmetrisch geformt ist oder sind.

13. Injektionsvorrichtung nach einem der vorhergehenden Ansprüche, wobei die Rutschkupplung eine Kupplungsfeder (9) zusätzlich zu dem Federglied (32) umfasst und die Kupplungsfeder (9) einem Lösen des Rasteingriffs eine rückstellende Federkraft entgegensetzt.

14. Injektionsvorrichtung nach dem vorhergehenden Anspruch, wobei die Rutschkupplung eine erste Kupplungshälfte (3) mit dem Rastelement (28) und eine zweite Kupplungshälfte (27) mit dem Rastgegenelement (28) umfasst und an wenigstens einer der Kupplungshälften (3, 27) die Kupplungsfeder (9) und an der Kupplungsfeder (9) entweder das Rastelement (28) oder das Rastgegenelement (28) geformt oder befestigt ist.

15. Injektionsvorrichtung nach dem vorhergehenden Anspruch, wobei eine der Kupplungshälften (3, 27) relativ zu der anderen um eine Drehachse (RT) drehbeweglich ist und die Kupplungsfeder (9) eine elastisch, vorzugsweise radial nachgiebige Biegefeder ist.

16. Injektionsvorrichtung nach dem vorhergehenden Anspruch, wobei die Kupplungsfeder (9) an der wenigstens einen der Kupplungshälften (3, 27) als Biegefeder geformt und an beiden Biegefederenden fest eingespannt ist.

17. Injektionsvorrichtung nach einem der vorhergehenden Ansprüche, wobei mehrere Rastelemente (28) oder Rastgegenelemente (8) vorgesehen sind und pro Rastelement (28) oder Rastgegenelement (8) vorzugsweise je eine eigene Kupplungsfeder (9) vorgesehen ist.

18. Injektionsvorrichtung nach einem der fünf vorhergehenden Ansprüche, wobei die wenigstens eine der Kupplungshälften (3, 27) einen Ring oder Teilring mit in Umfangsrichtung hintereinander angeordneten Ringsegmenten umfasst, von denen eines die Kupplungsfeder (9) und das andere ein demgegenüber steiferes Ringsegment bildet.

19. Injektionsvorrichtung nach dem vorhergehenden Anspruch, wobei der Ring oder Teilring in Umfangsrichtung hintereinander alternierend angeordnete elastische erste Ringsegmente und demgegenüber steifere zweite Ringsegmente umfasst und die ersten Ringsegmente je eine Kupplungsfeder (9) bilden.

20. Injektionsvorrichtung nach einem der vorhergehenden Ansprüche, wobei die Rutschkupplung (3, 27) mehrere Rastelemente (28) oder mehrere Rastgegenelemente (8) umfasst, die in dem Rasteingriff miteinander verrastend relativ zueinander in eine die Dosis vergrößernde Kupplungsdosierrichtung und eine entgegengerichtete Kupplungskorrekturrichtung beweglich sind.

21. Injektionsvorrichtung nach einem der vorhergehenden Ansprüche, wobei der Betätigungsknopf (27) für die Einstellung der Dosis mit einem Dosierglied (30) gekoppelt ist und das Dosierglied (30) bei der Ausschüttung der Dosis bis gegen einen Ausschüttanschlag (2c) und für die Einstellung der Dosis gegen die Federkraft des Federglieds (32) von dem Ausschüttanschlag (2c) weg beweglich ist.

22. Injektionsvorrichtung nach dem vorhergehenden Anspruch, wobei der Betätigungsknopf (27) und das Dosierglied (30) mittels einer Dosierkupplung (16, 22) miteinander lösbar gekoppelt sind.

23. Injektionsvorrichtung nach einem der zwei vorhergehenden Ansprüche, wobei das Dosierglied (30) bei der Einstellung der Dosis von dem Förderglied (10) entkoppelt und für die Ausschüttung mittels einer Ausschüttkupplung (12, 16) mit dem Förderglied (10) koppelbar ist.

24. Injektionsvorrichtung nach einem der vorhergehenden Ansprüche, wobei das Rastelement (28) an dem Betätigungsknopf (27) geformt oder befestigt ist.

25. Injektionsvorrichtung nach einem der vorhergehenden Ansprüche, wobei das Rastgegenelement (8) an dem Gehäuse (1-3) geformt oder befestigt ist.

26. Injektionsvorrichtung nach einem der Ansprüche 1-24, wobei das Rastgegenelement an einem relativ zu dem Gehäuse axial beweglichen, verdrehgesichert geführten Glied, an dem sich das Federglied abstützt, geformt oder befestigt ist.

27. Injektionsvorrichtung nach einem der vorhergehenden Ansprüche, wobei der Betätigungsknopf (27) für die Einstellung der Dosis relativ zu dem Gehäuse (1-3) rotatorisch beweglich ist.

28. Injektionsvorrichtung nach einem der vorhergehenden Ansprüche, wobei der Betätigungsknopf (27) einen Auslöser für die Ausschüttung bildend relativ zu dem Gehäuse (1-3) translatorisch beweglich ist, vorzugsweise in eine distale Richtung.

## Claims

1. Injection device comprising:
a) a housing (1-3) with a reservoir (R) for an injectable product,
b) an actuation knob (27) which, for the setting of a product dose can be moved relative to the housing (1-3) in a direction, which increases the dose and in a direction providing a correction of the dose,
c) a delivery element (10) for the emptying of the set dose,
d) a slipping clutch (3, 27) with a detent element (28) and a counter detent element (8), which come into mutual positive and non-positive engagement in discrete latching positions of the housing (1-3) to arrest the actuating head (27) during a movement in the dosing direction or in the correction direction.
e) and a spring element (32), which applies a spring force opposing the movement of the actuating head (27) in at least one of the directions,
f) **characterised in that** the detent element (28) and the counter detent element (8) are formed in such a way, that in the latching engagement of the motion they at least apply a lower resistance to the motion in one of the directions than in the other.

2. Injection device according to claim 1, in which the spring element (32) is coupled with, or can be coupled with the delivery element (10) and drives the spring element (32) during the emptying.

3. Injection device according to the foregoing claim, in which the spring element (32) is uncoupled from the delivery element (10) during the setting of the dose and can be coupled with the delivery element (10) for the emptying by means of an emptying coupling (12, 16).

4. Injection device according to one of the foregoing claims, in which during the movement of the actuating knob (27) the spring element (32) is more strongly tensioned in at least one of the directions from latched position to latched position.

5. Injection device according to one of the foregoing claims, in which the spring force of the spring element (32) opposes the movement in the direction of dosing.

6. Injection device according to one of the foregoing claims, in which the spring element (32) is a compression spring or a torsion spring.

7. Injection device according to one of the foregoing claims, in which the slipping clutch comprises a first clutch half (27) with the detent element (28) and a second clutch half (3) with the counter detent element (8) and one of the clutch halves (3, 27) can be rotated relative to the other about an axis of rotation (RT).

8. Injection device according to claim 7, in which the detent element (28) and the counter detent element (8) come into radial meshing engagement with each other with respect to the axis of rotation (RT).

9. Injection device according to claim 7, in which the detent element and the counter detent element come into axial meshing engagement with respect to the axis of rotation (RT).

10. Injection device according to one of the foregoing claims, in which the slipping clutch comprises a first clutch half (27) with the detent element (28) and a second clutch half (3) with the counter detent element (8) and the detent element (28) and the counter detent element (8) are in direct meshing engagement with each other.

11. Injection device according to one of the four last foregoing claims, in which one of the clutch halves (3, 27) can move relative to the other in the dosing device and relative to the correction direction.

12. Injection device according to one of the foregoing claims, in which the detent element (28) and/or the counter detent element (8) is/are formed asymmetrically with respect to the dosing device.

13. Injection device according to one of the foregoing claims, in which the slipping clutch comprises a clutch spring (9) in addition to the spring element (32) and the clutch spring (9) opposes a freeing of the meshing engagement by means of a resetting spring force.

14. Injection device according to claim 13, in which the slipping clutch comprises a first clutch half (3) with the detent element (28) and a second clutch half (27) with the counter detent element (8) and on at least one of the clutch halves (3, 27) the clutch spring (9), and on the clutch spring (9) either the detent element (28) or the counter detent element (8) is formed or fixed.

15. Injection device according to claim 14, in which one of the clutch halves (3, 27) can rotate relative to the other half about an axis of rotation (RT) and the clutch spring (9) is an elastic, preferably radial, spiral spring.

16. Injection device according to claim 15, in which the clutch spring (9) takes the form of a spiral spring on at least one of the clutch halves (3, 27) and is rigidly fixed to both in acting as a spiral spring.

17. Injection device according to one of the foregoing claims, in which a number of detent elements (28) or counter detent elements (8) are provided and preferably a clutch spring (9) per detent element (28) or counter detent element (8).

18. Injection device according to one of the five last foregoing claims, in which at least one of the clutch halves (3, 27) comprises a ring or part ring with ring segments arranged one behind the other in the circumferential direction, of which one forms the clutch spring (9) and the other an opposite stiffer ring element.

19. Injection device according to claim 18, in which the ring or part ring comprises elastic first ring segments and opposite stiffer second ring segments arranged alternately one behind the other and the first ring segments each form a clutch spring (9).

20. Injection device according to one of the foregoing claims, in which the slipping clutch (3, 27) comprises a number of detent elements (28) or a number of counter detent elements (8), which in engagement with one another can move relative to one another in a clutch dosing direction, in which the dose is increased and also in an opposite clutch dosing correction direction.

21. Injection device according to one of the foregoing claims, in which the actuation knob (27) for the setting of the dose is coupled with a dosing element (30) and the dosing element (30) can move during the emptying of the dose up to an emptying stop (2c) and for the setting of the dose away from the emptying stop (2c) against the spring force of the spring element (32).

22. Injection device according to claim 21, in which the actuation knob (27) and the dosing element (30) are detachably coupled together by means of a dosing clutch (16, 22).

23. Injection device according to claim 21 or claim 22, in which the dosing element (30) is uncoupled from the delivery element (10) for the setting of the dose and can be coupled with the delivery element (10) for the emptying by means of an emptying clutch (12, 16).

24. Injection device according to one of the foregoing claims, in which the detent element (28) is formed on or fixed to the actuating knob (27).

25. Injection device according to one of the foregoing claims, in which the counter detent element (8) is formed on or fixed to the housing (1-3).

26. Injection device according to one of the claims 1 24, in which the counter detent element is formed on or fixed to an element, which is guided to move axially relative to the housing while being prevented from twisting and on which the spring element is supported.

27. Injection device according to one of the foregoing claims, in which the actuating knob (27) can move in a rotary sense relative to the housing (1-3) for the setting of the dose.

28. Injection device according to one of the foregoing claims, in which the actuating knob (27) can describe a translatory motion relative to the housing (1-3), preferably in a distal direction, as a tripping device for the emptying.

## Revendications

1. Dispositif d'injection comportant
a) un boîtier (1-3) pourvu d'un réservoir (R) pour un produit injectable,
b) un bouton d'actionnement (27), lequel est mobile par rapport au boîtier (1-3) dans un sens de dosage augmentant la dose et un sens de correction, pour le réglage d'une dose de produit,
c) un élément de transport (10) pour la distribution de la dose réglée,
d) un accouplement à glissement (3, 27) muni d'un élément d'arrêt (28) et d'un contre-élément d'arrêt (8), qui accouplent, par complémentarité de forme et de force, en les mettant en prise d'arrêt le bouton d'actionnement (27) lors de son mouvement dans le sens de dosage ou de correction et le boîtier (1-3) respectivement en positions d'arrêt discrètes,
e) et un élément à ressort (32), qui oppose une force de ressort au mouvement du bouton d'actionnement (27) dans au moins une des directions,
**caractérisé en ce que** l'élément d'arrêt (28) ou le contre-élément d'arrêt (8) est formé de telle sorte que, en prise d'arrêt du mouvement dans au moins une des directions, ils opposent une résistance inférieure par rapport au mouvement dans l'autre direction.

2. Dispositif d'injection selon la revendication 1, l'élément à ressort (32) étant accouplé ou pouvant être accouplé à l'élément de transport (10) et entraînant l'élément de transport (32) lors de la distribution.

3. Dispositif d'injection selon la revendication précédente, l'élément à ressort (32) étant désaccouplé de l'élément de transport (10) lors du réglage de la dose et pouvant être accouplé à l'élément de transport (10) au moyen d'un accouplement de distribution (12, 16) pour la distribution.

4. Dispositif d'injection selon l'une quelconque des revendications précédentes, l'élément à ressort (32) étant tendu de plus en plus fortement, lors du mouvement du bouton d'actionnement (27), dans au moins une des directions d'une position d'arrêt à une autre.

5. Dispositif d'injection selon l'une quelconque des revendications précédentes, l'élément à ressort (32) opposant la force de ressort au mouvement dans le sens de dosage.

6. Dispositif d'injection selon l'une quelconque des revendications précédentes, l'élément à ressort (32) étant un ressort de pression ou un ressort de torsion.

7. Dispositif d'injection selon l'une quelconque des revendications précédentes, l'accouplement à glissement comportant une première moitié d'accouplement (27) munie de l'élément d'arrêt (28) et une seconde moitié d'accouplement (3) munie du contre-élément d'arrêt (8), et une des moitiés d'accouplement (3, 27) étant mobile par rotation par rapport à l'autre autour d'un axe de rotation (RT).

8. Dispositif d'injection selon la revendication précédente, l'élément d'arrêt (28) et le contre-élément d'arrêt (8) s'engrenant radialement l'un dans l'autre en prise d'arrêt par rapport à l'axe de rotation (RT).

9. Dispositif d'injection selon la revendication 7, l'élément d'arrêt et le contre-élément d'arrêt s'engrenant axialement l'un dans l'autre en prise d'arrêt par rapport à l'axe de rotation (RT).

10. Dispositif d'injection selon l'une quelconque des revendications précédentes, l'accouplement à glissement se composant d'une première moitié d'accouplement (27) munie de l'élément d'arrêt (28) et d'une seconde moitié d'accouplement (3) munie du contre-élément d'arrêt (8), et l'élément d'arrêt (28) et le contre-élément d'arrêt (8) étant directement en prise d'arrêt l'un avec l'autre.

11. Dispositif d'injection selon l'une quelconque des quatre revendications précédentes, une des moitiés d'accouplement (3, 27) étant mobile par rapport à l'autre dans le sens de dosage et le sens de correction.

12. Dispositif d'injection selon l'une quelconque des revendications précédentes, l'élément d'arrêt (28) ou le contre-élément d'arrêt (8) étant formé(s) de manière asymétrique par rapport au sens de dosage.

13. Dispositif d'injection selon l'une quelconque des revendications précédentes, l'accouplement à glissement comportant un ressort d'accouplement (9) en plus de l'élément à ressort (32), et le ressort d'accouplement (9) opposant une force de ressort de rappel à une libération de la prise d'arrêt.

14. Dispositif d'injection selon la revendication précédente, l'accouplement à glissement comportant une première moitié d'accouplement (3) munie de l'élément d'arrêt (28) et une seconde moitié d'accouplement (27) munie du contre-élément d'arrêt (8) et le ressort d'accouplement (9) étant formé ou fixé sur au moins une des moitiés d'accouplement (3, 27) et l'élément d'arrêt (28) ou le contre-élément d'arrêt (8) étant formé ou fixé sur le ressort d'accouplement (9).

15. Dispositif d'injection selon la revendication précédente, une des moitiés d'accouplement (3, 27) étant mobile par rotation par rapport à l'autre autour d'un axe de rotation (RT), et le ressort d'accouplement (9) étant un ressort de flexion élastique, de préférence flexible radialement.

16. Dispositif d'injection selon la revendication précédente, le ressort d'accouplement (9) étant formé sur au moins une des moitiés d'accouplement (3, 27) en tant que ressort de flexion et étant encastré fixement sur les deux extrémités du ressort de flexion.

17. Dispositif d'injection selon l'une quelconque des revendications précédentes, plusieurs éléments d'arrêt (28) ou contre-éléments d'arrêt (8) étant prévus, et de préférence un ressort d'accouplement (9) propre étant prévu par élément d'arrêt (28) ou contre-élément d'arrêt (8).

18. Dispositif d'injection selon l'une quelconque des cinq revendications précédentes, au moins une des moitiés d'accouplement (3, 27) comportant une bague ou une bague partielle avec des segments de bague disposés les uns derrière les autres dans le sens de la périphérie, dont un forme le ressort d'accouplement (9) et l'autre un segment de bague plus rigide par rapport à ce dernier.

19. Dispositif d'injection selon la revendication précédente, la bague ou la bague partielle comportant, dans le sens de la périphérie, des premiers segments de bague élastiques disposés de manière alternée les uns derrière les autres et des seconds segments de bague plus rigides par rapport à ces derniers, et les premiers segments de bague formant chacun un ressort d'accouplement (9).

20. Dispositif d'injection selon l'une quelconque des revendications précédentes, l'accouplement de glissement (3, 27) comportant plusieurs éléments d'arrêt (28) ou plusieurs contre-éléments d'arrêt (8), lesquels sont mobiles les uns par rapport aux autres, en prise d'arrêt mutuel, dans un sens de dosage d'accouplement augmentant la dose et un sens de correction d'accouplement opposé.

21. Dispositif d'injection selon l'une quelconque des revendications précédentes, le bouton d'actionnement (27) pour le réglage de la dose étant accouplé à un élément de dosage (30) et l'élément de dosage (30) étant mobile lors de la distribution de la dose jusqu'à une butée de distribution (2c) et pour le réglage de la dose contre la force de ressort de l'élément de ressort (32) depuis la butée de distribution (2c).

22. Dispositif d'injection selon la revendication précédente, le bouton d'actionnement (27) et l'élément de dosage (30) étant accouplés l'un à l'autre de manière amovible au moyen d'un accouplement de dosage (16, 22).

23. Dispositif d'injection selon l'une quelconque des deux revendications précédentes, l'élément de dosage (30) étant désaccouplé de l'élément de transport (10) lors du réglage de la dose et pouvant être accouplé à l'élément de transport (10) pour la distribution au moyen d'un accouplement de distribution (12, 16).

24. Dispositif d'injection selon l'une quelconque des revendications précédentes, l'élément d'arrêt (28) étant formé sur le bouton d'actionnement (27) ou fixé à ce dernier.

25. Dispositif d'injection selon l'une quelconque des revendications précédentes, l'élément d'arrêt (8) étant formé sur le boîtier (1-3) ou fixé à ce dernier.

26. Dispositif d'injection selon l'une quelconque des revendications 1-24, le contre-élément d'arrêt étant formé sur ou fixé à un élément guidé de manière bloquée en rotation, axialement mobile par rapport au boîtier, sur lequel s'appuie l'élément de ressort.

27. Dispositif d'injection selon l'une quelconque des revendications précédentes, le bouton d'actionnement (27) pour le réglage de la dose étant mobile par rotation par rapport au boîtier (1-3).

28. Dispositif d'injection selon l'une quelconque des revendications précédentes, le bouton d'actionnement (27), formant un déclencheur pour la distribution, étant mobile par rapport au boîtier (1-3), de préférence dans une direction radiale.
